Europäisches Patentamt

(19) European Patent Office

Office européen des brevets

(11) Numéro de publication: **0 204 596**
**B1**

(12) FASCICULE DE BREVET EUROPEEN

(45) Date de publication du fascicule du brevet:
09.08.89 ·

(21) Numéro de dépôt: 86400996.4

㉒ Date de dépôt. 09.05.86

(51) Int. Cl.⁴: **A61K 9/22, A61K 9/16, A61K 9/52**

ERRATUM

(SEITE, SPALTE, ZEILE)
(PAGE, COLUMN, LINE)
(PAGE, COLONNE, LIGNE)

| DIE TEXTSTELLE :<br>TEXT PUBLISHED :<br>LE PASSAGE SUIVANT : | | | | LAUTET BERICHTIGT:<br>SHOULD READ :<br>DEVRAIT ETRE LU : |
|---|---|---|---|---|
| d'alcools de C@$ à C#^ | 2 | | 43 | d'alcools de C24 à C36 |

| Tag der Entscheidung )<br>über die Berichtigung )<br>Date of decision on )<br>rectification: )<br>Date de décision portant )<br>sur modification: ) | 19.10.89 | Ausgabe- und Ver- )<br>öffentlichungstag: )<br>Issue and publication )<br>date: )<br>Date d'edition et de )<br>publication: · ) | 03.01.90 | Patbl.Nr)<br>90/01<br>EPB no: ) ........<br>Bull. no:) |

**Europäisches Patentamt**

**European Patent Office**

**Office européen des brevets**

(19)

(11) Numéro de publication: **0 204 596 B1**

## FASCICULE DE BREVET EUROPEEN

(12)

(45) Date de publication du fascicule du brevet:
09.08.89

(51) Int. Cl.⁴: **A61K 9/22**, A61K 9/16, A61K 9/52

(21) Numéro de dépôt: **86400996.4**

(22) Date de dépôt: **09.05.86**

(54) **Compositions pour la préparation par extrusion de microparticules permettant la libération prolongée d'une substance biologiquement active et les microparticules ainsi obtenues.**

(30) Priorité: 09.05.85 FR 8507013

(43) Date de publication de la demande
10.12.86 Bulletin 86/50

(45) Mention de la délivrance du brevet:
09.08.89 Bulletin 89/32

(84) Etats contractants désignés:
AT BE CH DE FR GB IT LI LU NL SE

(56) Documents cités:
EP-A- 0 043 254
US-A- 3 965 256

**Le dossier contient des informations techniques présentées postérieurement au dépôt de la demande et ne figurant pas dans le présent fascicule.**

(73) Titulaire: **RHONE-POULENC SANTE, 20, avenue Raymond Aron, F-92160 Antony(FR)**

(72) Inventeur. **Drouin, Jehan-Yves, 4 rue du Mérou. F-92290 Chatenay Malabry(FR)**
Inventeur: **Veillard, Michel, 12 rue du Docteur Roux, F-92330 Sceaux(FR)**

(74) Mandataire: **Pilard, Jacques et al, RHONE-POULENC RECHERCHES Service Brevets Pharma 25, Quai Paul Doumer, F-92408 Courbevoie Cedex(FR)**

## Description

La présente invention concerne de nouvelles compositions pour la préparation par extrusion de microparticules permettant la libération prolongée d'une substance biologiquement active et les microparticules ainsi obtenues.

Il existe différents procédés de préparation de microgranules permettant la libération prolongée d'une substance active. Par exemple, un procédé consiste à réaliser les étapes successives suivantes: montage d'un germe saccharose-amidon, imprégnation ou grossissage du germe par la substance active en poudre ou en solution, puis enrobage par des solutions de polymères qui confèrent la cinétique de libération désirée de la substance active. Un autre procédé comporte la réalisation des étapes successives suivantes: extrusion d'un mélange humide contenant la substance active, sphéronisation de l'extrudat, puis enrobage de la microsphère obtenue par des solutions de polymères qui confèrent la cinétique de libération de la substance active. Cependant ces techniques sont longues et coûteuses.

Par ailleurs, il arrive fréquemment que, en particulier par extrusion, on obtienne des particules de petite taille qui ont une surface d'échange importante ce qui conduit à une libération relativement rapide de la substance active. Pour ralentir la vitesse de libération, il est possible d'effectuer un enrobage, après une sphéronisation préalable, afin d'obtenir une membrane filmogène d'épaisseur connue.

Dans le brevet européen EP 43 254 sont décrites des compositions lipidiques destinées à préparer des comprimés permettant la libération contrôlée d'une substance active. Ces compositions peuvent contenir des polymères hydrophiles tels que la carboxyméthylcellulose qui jouent le rôle d'agent de désintégration ou de gonflement.

Dans le brevet américain US 3 965 256 sont décrits des comprimés permettant la libération contrôlée d'une substance active constitués de la substance active, d'un alcool gras et d'un polymère hydrophile tel que les hydroxyalkylcelluloses.

Il a maintenant été trouvé, et c'est ce qui fait l'objet de la présente invention, qu'une composition constituée d'une substance active, d'un ou plusieurs polymères non hydrophiles et de 10 à 40% en poids soit d'un mélange d'au moins deux excipients lipidiques dont l'un possède la propriété de dissoudre ou gélifier le ou les polymères et l'autre possède des propriétés lubrifiantes soit d'un excipient lipidique possédant à la fois la propriété de dissoudre ou gélifier le ou les polymères et des propriétés lubrifiantes, et éventuellement d'un ou plusieurs additifs choisis parmi les diluants et les agents antistatiques, peut être extrudée pour fournir des microparticules permettant la libération prolongée de la substance active.

Les polymères qui sont utilisés pour la réalisation des microparticules selon l'invention sont choisis parmi les éthers cellulosiques (telles que les éthylcelluloses des séries G, K, N et T, et plus particulièrement celles de la série N, N.D. Hercules), les polymères d'esters d'acides acryliques et méthacryliques (tels que Eudragit® RSPM, RLPM, L et S et plus particulièrement RSPM, N.D. Röhmpharma), les copolymères de vinylpyrrolidone et d'acétate de vinyle (tel que le Kollidon® VA 64, N.D. B.A.S.F.), les alcools polyvinyliques comme les Mowiols® (N.D. Hoechst) et les homopolymères de l'acétate de vinyle comme par exemple le Rhodopas® BB 3 (N.D. Rhône-Poulenc).

Le choix du ou des polymères est effectué en fonction de l'affinité de la substance active pour les milieux aqueux. Ainsi, d'une manière générale, dans le cas d'une substance active hydrophile, compte tenu de la petite taille des microparticules et de leur grande surface d'échange, il est particulièrement avantageux d'utiliser un polymère non hydrophile et non érodible (tel que l'éthylcellulose de type N) pour obtenir une libération prolongée sur plus de 8 heures par voie orale chez l'homme. En revanche, il est particulièrement avantageux d'utiliser un polymère érodible, c'est-à-dire un polymère qui est lentement soluble dans l'eau et/ou progressivement digestible par les enzymes présents dans les liquides biologiques (tel qu'un copolymère de vinylpyrrolidone et d'acétate de vinyle comme le Kollidon® VA 64, N.D. BASF), pour obtenir une libération totale de la substance active en 24 heures.

L'association de 2 ou plusieurs polymères constitue un moyen supplémentaire de contrôle de la cinétique de libération en fonction des caractéristiques propres de la substance active. Il peut aussi être avantageux, dans certains cas, de contrôler la cinétique de libération par addition de polymères particulièrement hydrophobes tels que les polysiloxanes.

Les excipients lipidiques sont choisis parmi les alcools gras (alcool cétylique), les acides gras (acide stéarique), les esters d'alcools de $C_{@\$}$ à $C_{\#^}$ et d'acides gras pouvant avoir 36 atomes de carbone (cire blanche), les huiles végétales polyoxyéthylénées (Cremophors® N.D. BASF; Labrafils®, N.D. Gattefossé), les huiles végétales hydrogénées (Cutina H.R., N.D. Henkel), les mono-, di- ou triglycérides d'acides gras (Compritol® 888 ou Precirol®, N.D. Gattefossé; Imwitor® 900 ou Softisan® 154, N.D. Dynamit Nobel) ou les lécithines.

Le ou les excipients lipidiques doivent posséder un pouvoir solubilisant ou gélifiant vis-à-vis du polymère et un pouvoir lubrifiant permettant l'extrusion.

Il est particulièrement avantageux de réaliser une composition contenant un excipient lipidique dont le point de fusion est voisin de 50°C pour dissoudre ou gélifier le polymère associé à un second excipient lipidique de point de fusion plus élevé pour favoriser la lubrification.

Il est possible d'utiliser un excipient lipidique unique, tel que le palmito-stéarate de glycérol (Precirol® N.D. Gattefossé) lorsque celui-ci associe un point de fusion peu élevé à des propriétés lubrifiantes convenables et lorsqu'il possède la propriété de dissoudre ou gélifier le polymère.

Dans les compositions extrudables selon la présente invention, la teneur en excipient lipidique représente généralement entre 10 et 40% en poids de la composition et il est particulièrement avantageux d'utiliser un mélange d'excipients lipidiques dans lequel l'excipient lubrifiant représente de 60 à 80% en poids du mélange.

Dans les compositions extrudables selon la présente invention, la substance biologiquement active représente généralement de 5 à 40% en poids de la composition.

La vitesse de libération de la substance active est influencée par la taille des microparticules, la nature et la quantité d'excipient lipidique et l'affinité de la substance active vis-à-vis de l'excipient lipidique.

Généralement, la vitesse de libération de la substance active augmente lorsque la taille des microparticules diminue, cette diminution de taille s'accompagnant d'un accroissement de la surface d'échange.

La vitesse de libération est fonction de l'affinité comparée de la substance active vis-à-vis, d'une part, des excipients lipidiques constituant les microparticules, et d'autre part, des milieux aqueux dans lesquels la substance active est libérée et elle est également fonction de la vitesse de diffusion de la substance active dans la matrice qui est liée à la nature et à la quantité du ou des polymères. Il en résulte qu'une substance active lipophile, telle que le kétoprofène, diffusera moins vite dans un excipient lipidique pour lequel la substance active a une plus grande affinité. Inversement, une substance active moins liphophile, telle que la riodipine ou le chlorhydrate d'acébutolol, diffusera plus vite dans un excipient lipidique pour lequel la substance active a moins d'affinité.

Pour la réalisation des microparticules selon l'invention, il est préférable d'utiliser un excipient lipidique ou un mélange d'excipients lipidiques dans lequel le polymère qui joue le rôle d'agent structurant est soluble ou partiellement soluble.

Selon la présente invention, les microparticules sont obtenues par un procédé d'extrusion qui consiste à faire passer un granulé homogène constitué du mélange d'un ou plusieurs polymères et d'un ou plusieurs excipients lipidiques contenant la substance active à travers les perforations calibrées.

La granulation peut être effectuée dans un granulateur classique en utilisant comme liquide de mouillage le ou les excipients lipidiques fondus ou bien dans un appareil à double enveloppe chauffante muni d'un couteau rotatif et d'un racleur en élevant progressivement la température jusqu'à l'entrée en fusion des excipients lipidiques afin de provoquer la granulation. En opérant de cette manière, il est possible d'obtenir un granulé dont l'homogénéité est beaucoup plus grande qu'en opérant dans un granulateur classique.

Selon la texture du granulé obtenu, il peut être nécessaire d'effectuer, préalablement à l'extrusion, une opération de régularisation dans le but de briser les amas.

L'extrusion peut être avantageusement réalisée dans un appareil constitué essentiellement de deux rouleaux tournant en sens inverse, l'un étant plein, l'autre étant perforé. Le granulé entraîné sous forte pression entre les deux rouleaux est extrudé à travers le rouleau perforé sous forme de petits cylindres de diamètres sensiblement identiques et dont la longueur est quasiment constante grâce à un couteau qui arrase l'extrudat à la sortie des pores. Les extrudats ainsi obtenus peuvent être tamisés afin de conserver un ensemble homogène.

L'extrusion du granulé est rendue possible grâce à l'augmentation de température qui se produit entre les deux rouleaux. Cette élévation de température conduit à la fusion partielle de l'excipient lipidique dont le point de fusion est le plus bas et qui dissout partiellement le polymère en donnant naissance à une masse plastique qui est extrudée et qui se resolidifie immédiatement.

Il est donc particulièrement important de contrôler la température d'extrusion. Ce contrôle peut être avantageusement effectué en agissant sur le débit d'alimentation en granulé et/ou sur la vitesse de rotation des rouleaux de telle manière que l'énergie thermique soit entièrement absorbée par le granulé pénétrant entre les deux rouleaux.

Il en résulte que, si l'apport calorique est trop faible, du fait par exemple d'une vitesse de rotation trop lente des rouleaux, l'extrusion ne sera que partielle et il sera nécessaire de recycler une partie importante du granulé et, si l'apport calorique est trop élevé, du fait par exemple d'une vitesse de rotation trop rapide des rouleaux, l'excédent calorique ne peut pas être absorbé par le granulé avant l'extrusion, ce qui conduit à une augmentation de température entraînant une fusion plus importante de l'excipient lipidique et par suite un colmatage rendant ainsi l'extrusion de plus en plus difficile.

Il est particulièrement avantageux d'effectuer l'extrusion à travers des orifices dont le diamètre est voisin de 1,5 mm.

Les microparticules obtenues par extrusion des compositions selon l'invention se présentent généralement sous forme de bâtonnets cylindriques dont la longueur est comprise entre 1 et 5 mm et dont le diamètre est compris entre 1 et 1,5 mm.

Les microparticules selon la présente invention peuvent être, par exemple, réparties uniformément dans des gélules. Selon le type de gélules utilisées, les microparticules pourront éventuellement subir un traitement de régularisation afin de rendre leur forme et leur taille compatibles avec un remplissage régulier.

Il est possible également de remplir les gélules avec un mélange de microparticules dont les cinétiques de dissolution sont différentes.

Les exemples suivants, donnés à titre non limitatif, montrent comment l'invention peut être mise en pratique.

## Exemple 1

A 34 g d'alcool cétylique fondu à une température de 65° C on ajoute 10 g de kétoprofène.

La solution ainsi obtenue est ajoutée, par petites fractions, à 56 g d'éthylcellulose N4 placés dans un mélangeur planétaire type "Bouvard" (N.D.). La vitesse d'agitation est de 50 tours/minute. L'agitation est poursuivie pendant 10 minutes jusqu'à l'obtention d'un granulé homogène.

Le granulé ainsi obtenu est extrudé dans un extrudeur type Alexander Werk (N.D.) dont les perforations du rouleau perforé ont un diamètre de 1 mm.

On obtient ainsi des microgranules qui se présentent sous forme de bâtonnets dont le diamètre est compris entre 1 et 1,25 mm et dont la longueur est comprise entre 1 et 5 mm.

## Exemples 2 à 8

On opère comme dans l'exemple 1 en remplaçant l'alcool cétylique par divers excipients lipidiques.
Les résultats obtenus sont rassemblés dans le tableau 1.

Tableau 1

| Exemples | Excipients lipidiques | Caractéristiques |
|---|---|---|
| 2 | Acide stéarique | Petits bâtonnets |
| 3 | Cire blanche | dont le diamètre |
| 4 | Imwitor® 900 | est compris entre |
| 5 | Cutina® HR | 1 et 1,25 mm et |
| 6 | Précirol® | dont la longueur est comprise entre |
| 7 | Compritol® 8–88 | 1 et 5 mm |
| 8 | Softisan® 154 | |

## Exemples 9 à 17

On opère comme dans l'exemple 1 mais en faisant varier la nature et les proportions des excipients lipidiques et des polymères; la teneur en substance active (kétoprofène) étant de 10% du poids total de la composition.

Les résultats obtenus sont rassemblés dans le tableau 2.

Tableau 2

| Exemples | Excipient lipidique % | Polymère % | Caractéristiques |
|---|---|---|---|
| 9 | Alcool cétylique 15 | Ethylcellulose N4 75 | Petits bâtonnets dont le diamètre |
| 10 | Alcool cétylique 10 | Ethylcellulose N4 80 | est compris entre 1 et 1,25 mm et |
| 11 | Précirol® 34 | Ethylcellulose N4 56 | dont la longueur est comprise entre |
| 12 | Précirol® 15 | Ethylcellulose N4 75 | 1 et 5 mm |
| 13 | Précirol® 10 | Ethylcellulose N4 80 | |
| 14 | Précirol® 34 | Moviol® 4–88 56 | |
| 15 | Précirol® 34 | Kollidon® VA 64 56 | |
| 16 | Précirol® 34 | Rhodopas® BB 3 56 | |
| 17 | Précirol® 34 | Eudragit® RSPM 56 | |

La libération de la substance active en fonction du temps est déterminée de la manière suivante:

Les essais sont effectués dans un "dissolutest" type USP XX. Cet appareil est constitué d'un bain-marie réglé à 37°C contenant 6 réacteurs.

Chaque réacteur est rempli avec 750 cm3 d'un milieu dont le pH est égal à 7,4 ayant la composition suivante:

— phosphate disodique 1302 g
— acide citrique 40 g
— eau distillée q.s.p. 20 litres

Dans chaque réacteur on plonge un agitateur tournant à la vitesse de 120 tours/minute.

Une gélule contenant 200 mg de microparticules est placée dans chaque réacteur au temps t = 0.

Un prélèvement de 5 cm3 est effectué au bout de 30 minutes, 1 heure, 2 heures, 3 heures, 4 heures, 5 heures et 23 heures. On dose dans chaque prélèvement la quantité de substance active libérée. Dans le cas du kétoprofène, le dosage est effectué par spectrophotométrie à 260 nm.

Les résultats obtenues sont rassemblés dans le tableau 3.

Tableau 3

| Exemples | % de substance active libérée après | | | | | | | |
|----------|--------|-----|-----|-----|-----|-----|-----|------|
|          | 30 min | 1 h | 2 h | 3 h | 4 h | 5 h | 6 h | 23 h |
| 1  | 34 | 40 | 51 |    | 61 |    | 66 | 83  |
| 2  | 23 | 28 | 37 | 45 | 47 | 52 |    | 76  |
| 3  | 12 | 22 | 38 | 47 | 50 | 54 |    | 75  |
| 4  | 25 | 38 |    | 60 |    |    | 76 | 92  |
| 5  | 2  | 3  |    | 5  |    |    | 6  | 11  |
| 6  | 5  | 10 |    | 30 |    |    | 45 | 68  |
| 7  | 3  | 4  | 6  | 11 |    | 15 |    | 44  |
| 8  | 3  | 5  | 6  | 8  | 9  | 10 |    | 18  |
| 9  | 19 | 26 | 36 |    | 48 |    | 53 | 81  |
| 10 | 18 | 24 | 33 |    | 43 |    | 51 | 76  |
| 11 | 5  | 10 |    | 30 |    |    | 45 | 68  |
| 12 | 4  | 8  | 17 |    | 35 |    | 47 | 81  |
| 13 | 4  | 8  | 15 |    | 26 |    | 33 | 60  |
| 14 | 36 | 70 |    | 97 |    | 98 |    | 100 |
| 15 | 26 | 63 |    | 90 |    | 92 |    | 96  |
| 16 | 14 | 36 | 58 | 71 | 75 |    | 79 | 85  |
| 17 | 8  | 16 |    | 32 | 36 | 40 |    | 77  |

Exemple 18

Dans la cuve à double enveloppe d'un granulateur de type "OLZA" (N.D.) possédant un racleur et un couteau rotatif en fond de cuve, on introduit 60 g de riodipine, 165 g de copolymère vinylpyrrolidone-acétate de vinyle (Kollidon® VA 64), 18,75 g d'alcool cétylique, 56,25 g de Precirol®. La température du mélange agité est élevée progressivement par circulation d'eau chaude thermostatée à 65°C dans la double enveloppe. Il se forme un granulé qui est soutiré puis extrudé dans un extrudeur type "Alexander Werk".

On obtient ainsi des microparticules qui se présentent sous forme de bâtonnets dont le diamètre est voisin de 1,5 mm et dont la longueur est comprise entre 1 et 5 mm.

Exemples 19 à 30

On opère comme dans l'exemple 18 mais en utilisant des mélanges extrudables dont la composition est donnée dans le tableau 4.

Tableau 4

| No exemple | Principe actif % | Polymère % | Excipient lipidique % | |
|---|---|---|---|---|
| 19 | Kétoprofène 20 | Kollidon® VA 64 55 | Alcool cétylique Precirol® | 6,25 18,75 |
| 20 | Riodipine 20 | Kollidon® VA 64 65 | Alcool cétylique Precirol® | 3,75 11,25 |
| 21 | Riodipine 20 | Kollidon® VA 64 50 | Alcool cétylique Precirol® | 7,5 22,5 |
| 22 | Riodipine 20 | Kollidon® VA 64 55 | Alcool cétylique Cutina® HR | 6,25 18,75 |
| 23 | Riodipine 20 | Kollidon® VA 64 55 | Alcool cétylique Compritol® 8-88 | 6,25 18,75 |
| 24 | Kétoprofène 20 | Mowiol® 4-88 55 | Alcool cétylique Precirol® | 6,25 18,75 |
| 25 | Kétoprofène 20 | Rhodopas® BB 3 55 | Alcool cétylique Precirol® | 6,25 18,75 |
| 26 | Kétoprofène 20 | Kollidon® VA 64 54 Ethylcellulose N4 1 | Alcool cétylique Precirol® | 6,25 18,75 |
| 27 | Kétoprofène 20 | Kollidon® VA 64 45 Ethylcellulose N4 10 | Alcool cétylique Precirol® | 6,25 18,75 |
| 28 | Kétoprofène 20 | Kollidon® VA 64 25 Ethylcellulose N4 30 | Alcool cétylique Precirol® | 6,25 18,75 |
| 29 | Kétoprofène 20 | Kollidon® VA 64 45 Natrosol® 250 HHX 10 | Alcool cétylique Precirol® | 6,25 18,75 |
| 30 | Riodipine 20 | Kollidon® VA 64 45 Huile de silicone V 300 000 10 | Alcool cétylique Precirol® | 6,25 18,75 |

La libération de la substance active en fonction du temps, à partir des microparticules qui font l'objet des exemples 18 à 30, est déterminée de la manière suivante:

Les essais sont effectués dans un "dissolutest" type USP XX. Cet appareil est constitué d'un bain-marie réglé à 37°C contenant 6 réacteurs.

Chaque réacteur est rempli avec 1 litre de milieu de dissolution qui est constitué, dans le cas de la riodipine, d'une solution de Cremophor EL à 2% et, dans le cas du kétoprofène, d'une solution aqueuse de pH égal à 5 ayant la composition suivante:

— acide acétique: 205,9 g

— phosphate disodique dihydraté: 363,12 g

— eau déminéralisée q.s.p.: 20 litres

Dans chaque réacteur plonge un agitateur qui est, soit une pâle tournant à 100 tours/minute, soit un panier tournant à 120 tours/minute.

Une gélule contenant 200 mg de microparticules est placée dans le réacteur au temps t = 0.

Un prélèvement de 5 cm3 est effectué après 1 heure, 2 heures, 3 heures, 4 heures, 5 heures, 6 heures et 22 heures. La quantité de substance active libérée est dosée dans chaque prélèvement par spectrophotométrie à 260 nm dans le cas du kétoprofène et à 360 nm dans le cas de la riodipine.

Les résultats obtenus sont rassemblés dans le tableau 5.

Tableau 5

| Microparticules des exemples | % de substance active libérée après | | | | | | |
|---|---|---|---|---|---|---|---|
| | 1 h | 2 h | 3 h | 4 h | 5 h | 6 h | 22 h |
| 18 | 18 | 30 | 36 | 42 | 48 | 51 | 88 |
| | 12* | 20 | 23 | 27 | 29 | 32 | 53 |
| 19 | 22 | 33 | 62 | 75 | 85 | 90 | 100 |
| 20 | 8* | 18 | 25 | 28 | 32 | 36 | 65 |
| 21 | 12* | 22 | 27 | 30 | 33 | 36 | 58 |
| 22 | 12* | 17 | 23 | 25 | – | 32 | 69 |
| 23 | 14* | 25 | 32 | 37 | – | 45 | 85 |
| 24 | 35 | 60 | 72 | 78 | 84 | 90 | 95 |
| 25 | 15 | 23 | 32 | 35 | 42 | 44 | 68 |
| 26 | 18 | 50 | 62 | 70 | 78 | 80 | 98 |
| 27 | 25 | 43 | 52 | 59 | 64 | 69 | 95 |
| 28 | 14 | 23 | 29 | 34 | 39 | 42 | 70 |
| 29 | 33 | 54 | 74 | 88 | 93 | 95 | 102 |
| 30 | 7,5 | 14,5 | 20,3 | 27,0 | 33,0 | 38,8 | 92,2 |

* Essais dans lesquels l'agitation est effectuée dans un panier tournant à 120 tours/minute.

## Revendications

1. Composition pour la préparation par extrusion de microparticules permettant la libération prolongée d'une substance biologiquement active caractérisée en ce qu'elle est constituée d'une substance biologiquement active, d'un ou plusieurs polymères non hydrophiles et de 10 à 40% en poids soit d'un mélange d'au moins deux excipients lipidiques dont l'un possède la propriété de dissoudre ou gélifier le ou les polymères et l'autre possède des propriétés lubrifiantes soit d'un excipient lipidique possédant à la fois la propriété de dissoudre ou gélifier le ou les polymères et des propriétés lubrifiantes, et éventuellement d'un ou plusieurs additifs choisis parmi les diluants et les agents antistatiques.

2. Composition selon la revendication 1 caractérisée en ce que le polymère non hydrophile est choisi parmi les éthers cellulosiques non hydrophiles, les polymères d'esters d'acides acryliques et méthacryliques, les copolymères de vinylpyrrolidone et d'acétate de vinyle, les alcools polyvinyliques et les homopolymères de l'acétate de vinyle et leurs mélanges.

3. Composition selon la revendication 1 caractérisée en ce que l'excipient lipidique est choisi parmi les alcools gras, les acides gras, les esters d'alcools gras et d'acides gras, les huiles végétales hydrogénées, les huiles végétales polyoxyéthylénées, les mono-, di- ou triglycérides d'acides gras, les lécithines et leurs mélanges.

4. Composition selon la revendication 1 caractérisée en ce que la substance active représente 5 à 40% en poids de la composition.

5. Composition selon la revendication 1 caractérisée en ce que, lorsque l'on utilise un mélange d'excipients lipidiques, l'excipient lubrifiant représente 60 à 80% en poids du mélange.

6. Composition selon la revendication 1 caractérisée en ce qu'elle contient en outre un polymère très hydrophobe.

7. Composition selon la revendication 6 caractérisée en ce que le polymère très hydrophobe est choisi parmi les polysiloxanes.

8. Composition selon la revendication 1 caractérisée en ce que la substance active est le kétoprofène.

9. Microparticules permettant la libération prolongée d'une substance biologiquement active caractérisées en ce qu'elles sont obtenues par extrusion d'une composition selon la revendication 1.

## Patentansprüche

1. Zusammensetzung für die Herstellung von Mikropartikeln durch Extrusion für verzögerte Abgabe einer biologisch aktiven Substanz, dadurch gekennzeichnet, daß sie aus einer biologisch aktiven Substanz, einem oder mehreren nicht hydrophilen Polymeren und 10 bis 40 Gew.-% entweder einer Mischung von mindestens zwei Lipid-Bindemitteln, wovon das eine das oder die Polymere lösende oder gelierende Eigenschaften und das andere Schmiermitteleigenschaften besitzt, oder eines Lipid-Bindemittels, das jedenfalls das oder die Polymere lösende oder gelierende Eigenschaft und Schmiermitteleigen-

7

schaften besitzt, und gegebenenfalls einen Zusatz oder mehrere Zusätze, ausgewählt aus Streckmitteln und antistatischen Mitteln, besteht.

2. Zusammensetzung nach Anspruch 1, dadurch gekennzeichnet, daß das nicht hydrophile Polymer ausgewählt ist aus den nicht hydrophilen Celluloseäthern, den Polymeren von Acryl- und Methacrylsäureestern, den Copolymeren von Vinylpyrrolidon und Vinylacetat, den Polyvinylalkoholen und den Homopolymeren von Vinylacetat und deren Mischungen.

3. Zusammensetzung nach Anspruch 1, dadurch gekennzeichnet, daß das Lipid-Bindemittel ausgewählt ist aus den Fettalkoholen, Fettsäuren, den Estern von Fettalkoholen und Fettsäuren, den hydrierten Pflanzenölen, den polyoxäthylierten Pflanzenölen, den Mono-, Di- oder Tri-Glyceriden von Fettsäuren, den Lecithinen und deren Mischungen.

4. Zusammensetzung nach Anspruch 1, dadurch gekennzeichnet, daß die aktive Substanz 5 bis 40 Gew.-% der Zusammensetzung ausmacht.

5. Zusammensetzung nach Anspruch 1, dadurch gekennzeichnet, daß bei Verwendung einer Mischung von Lipid-Bindemitteln das Schmiermittel-Bindemittel 60 bis 80 Gew.-% der Mischung ausmacht.

6. Zusammensetzung nach Anspruch 1, dadurch gekennzeichnet, daß sie zusätzlich ein stark hydrophobes Polymer enthält.

7. Zusammensetzung nach Anspruch 6, dadurch gekennzeichnet, daß das stark hydrophobe Polymer ausgewählt ist aus den Polysiloxanen.

8. Zusammensetzung nach Anspruch 1, dadurch gekennzeichnet, daß die aktive Substanz Ketoprofen ist.

9. Mikropartikel für verzögerte Abgabe einer biologisch aktiven Substanz, dadurch gekennzeichnet, daß sie durch Extrusion einer Zusammensetzung nach Anspruch 1 hergestellt sind.

**Claims**

1. A composition for the preparation by extrusion of microparticles permitting a prolonged release of a biologically active substance, which composition consists of a biologically active substance, one or more non-hydrophilic polymers and from 10 to 40% by weight either of a mixture of at least two lipid excipients one of which has the property of dissolving or gelling the pollymer or polymers and the other has lubricating properties or of a lipid excipient having both the property of dissolving or gelling the polymer or polymers and lubricating properties, and, if appropriate, one or more additives chosen from diluents and antistatic agents.

2. A composition according to claim 1, wherein the non-hydrophilic polymer is chosen from non-hydrophilic cellulose ethers, polymers of esters of acrylic and methacrylic acids, copolymers of vinylpyrrolidone and vinyl acetate, polyvinyl alcohols and homopolymers of vinyl acetate and their mixtures.

3. A composition according to claim 1, wherein the lipid excipient is chosen from fatty alcohols, fatty acids, esters of fatty alcohols with fatty acids, hydrogenated vegetable oils, polyoxyethylenated vegetable oils, fatty acid mono-, di- or triglycerides, lecithins and their mixtures.

4. A composition according to claim 1, wherein the active substance represents 5 to 40% by weight of the composition.

5. A composition according to claim 1, wherein, when a mixture of lipid excipients is used, the lubricating excipient represents 60 to 80% by weight of the mixture.

6. A composition according to claim 1, which additionally contains a highly hydrophobic polymer.

7. A composition according to claim 6, wherein the highly hydrophobic polymer is chosen from polysiloxanes.

8. A composition according to claim 1, wherein the active substance is ketoprofen.

9. Microparticles permitting a prolonged release of a biologically active substance which are produced by extrusion of a composition according to claim 1.